(19) Europäisches Patentamt
European Patent Office
Office européen des brevets

(11) Veröffentlichungsnummer: **0 109 932**
**B1**

(12) # EUROPÄISCHE PATENTSCHRIFT

(45) Veröffentlichungstag der Patentschrift: **03.09.86**

(51) Int. Cl.⁴: **C 08 G 69/44**

(21) Anmeldenummer: **83810509.6**

(22) Anmeldetag: **04.11.83**

(54) **Verfahren zur Herstellung von oligomeren Polyesteramiden mit Seitengruppen enthaltend einen sterisch gehinderten Aminrest.**

(30) Priorität: **10.11.82 US 440490**

(43) Veröffentlichungstag der Anmeldung: **30.05.84 Patentblatt 84/22**

(45) Bekanntmachung des Hinweises auf die Patenterteilung: **03.09.86 Patentblatt 86/36**

(84) Benannte Vertragsstaaten: **BE DE FR GB IT NL**

(56) Entgegenhaltungen:
**DE-A-2 719 131**
**DE-A-3 111 739**

(73) Patentinhaber: **CIBA- GEIGY AG, Klybeckstrasse 141, CH- 4002 Basel (CH)**

(72) Erfinder: **Winter, Roland A.E., 23 Banksville Road, Armonk New York 10504 (US)**
Erfinder: **Malherbe, Roger, 17 Hillcrest Avenue, Yonkers New York 10705 (US)**
Erfinder: **Tieh- Yin Fu, Frank, 23 Tung Shan Street, Taipei 100 ROC (TW)**

**EP 0 109 932 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Verfahren zur Herstellung von polymeren Lichtschutzmitteln. Es handelt sich bei den Produkten um Oligoesteramide, die als Seitengruppe einen sterisch gehinderten Aminrest enthalten.

Es ist bekannt, dass 2,2,6,6-tetrasubstituierte Piperidinderivate wirkungsvolle Lichtschutzmittel für organische Materialien sind, insbesondere für organische Polymere wie z.B. für Polypropylen. Um die Flüchtigkeit solcher Lichtschutzmittel zu vermindern hat man polymere oder oligomere Verbindungen geschaffen, die solche Piperidinreste entweder in der Polymer-Hauptkette (z.B. DE-OS 27 19 131) oder als Seitengruppen enthalten (z.B. EP-A 496). In der DE-OS 31 11 739 wurden auch bereits oligomere Polyesteramide mit Polyalkylpiperidingruppen der Formel IX beschrieben

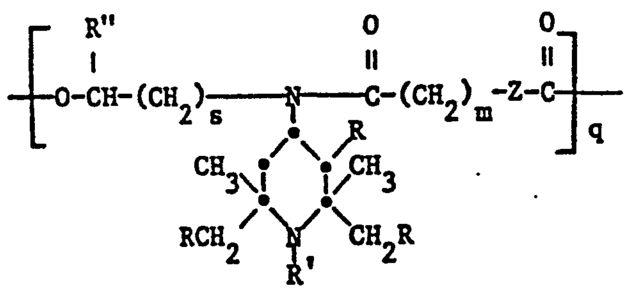

(IX),

worin R Wasserstoff oder $CH_3$, R' Wasserstoff, Oxyl-Sauerstoff, Alkyl, Hydroxyalkyl, Alkenyl, Propargyl, Benzyl oder Acetyl ist, m null oder 1 ist, s 1 oder 2 ist, q einen Wert von 2-50 darstellt, R'' Wasserstoff $CH_3$ oder $C_2H_5$ ist und Z eine Gruppe

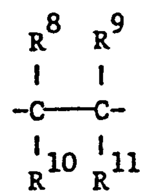

oder

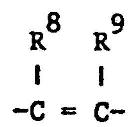

ist, worin $R^8$ H oder Alkyl und $R^9$ H, Alkyl, Alkenyl oder Phenyl bedeuten oder $R^8$ und $R^9$ zusammen mit den beiden C-Atomen, an die sie gebunden sind, einen gesättigten oder ungesättigten, mindestens 5-gliedrigen carbocyclischen Ring bilden, und $R^{10}$ und $R^{11}$ H oder $CH_3$ sind.

Als Beispiel Nr. 54 wird dort ein solches oligomeres Polyesteramid der Formel

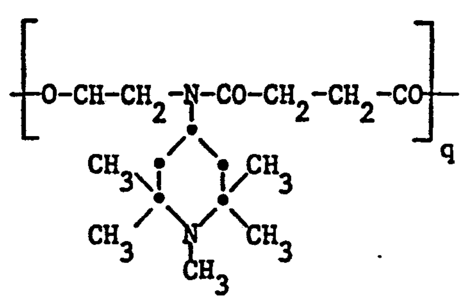

gebracht, das bei 90° erweicht und ein mittleres Molekulargewicht $\bar{M}n$ von 1900 hat.

Die Herstellung dieser Verbindungen der Formel IX erfolgt durch Umsetzung eines Aminoalkohols der Formel

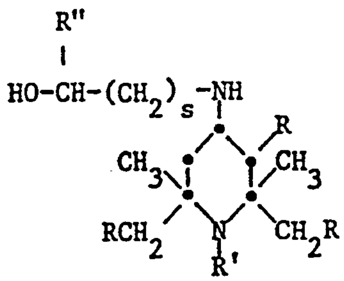

bei tiefer Temperatur mit einem Moläquivalent eines Dicarbonsäureanhydrids der Formel

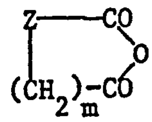

und anschliessender Veresterung der gebildeten Carboxylgruppe (z.B. mit Dimethylsulfat) und anschliessender Polykondensation des gebildeten Hydroxy-esters. Diese Herstellung verläuft also vom Aminoalkohol aus in drei Stufen.

Es wurde gefunden, dass solche und weitere strukturell ähnliche Verbindungen wesentlich einfacher hergestellt werden können durch Umsetzung eines Aminoalkohols der Formel 1

$$G - NH - L - OH \qquad (I)$$

mit einem Niederalkylester einer Dicarbonsäure der Formel II

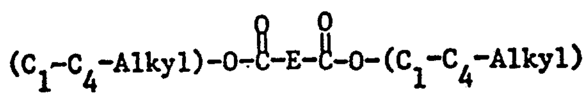 (II)

im annähernden Molverhältnis von 1:1 unter Bildung von oligomeren Polyesteramiden der Formel III.

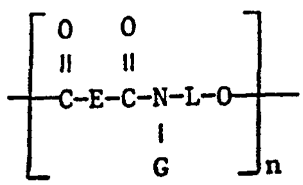 (III)

> erin bedeutet G eine Gruppe der Formel IV oder V,

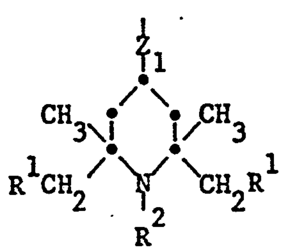

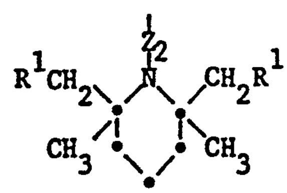

(IV) (V)

worin $R^1$ Wasserstoff oder $C_1$-$C_4$-Alkyl ist, $R_2$ Wasserstoff, $C_1$-$C_{12}$-Alkyl, Allyl, Benzyl, Acetyl oder Acryloyl ist, $Z_1$ eine direkte Bindung oder eine Gruppe $-NHCH_2CH_2-$, $-NH-(CH_2)_3-$ oder $-O-CH_2CH_2-$, deren Heteroatom an den Piperidinring gebunden ist, darstellt, und $Z_2$ $-(CH_2)_2-$ oder $-(CH_2)_3-$ darstellt, E bedeutet $C_2$-$C_{12}$-Alkylen, Phenylen, 5-Norbornen-2,3-diyl oder -CH=CH- und

L bedeutet $-(CH_2)_2-$, $-(CH_2)_3-$ oder $-CH_2-CH(R^3)-$, worin $R^3$ $C_1$-$C_6$-Alkyl, $C_5$-$C_6$-Cycloalkyl oder Phenyl ist und n

einen Wert von 2 bis 50, vorzugsweise 2 bis 10, darstellt.

$R^1$ als Alkyl kann z.B. Methyl, Ethyl, Isopropyl oder n-Butyl sein. Vorzugsweise ist $R^1$ Wasserstoff.

$R^2$ als Alkyl kann z.B. Methyl, Ethyl, Isopropyl, n-Butyl, n-Hexyl, 2-Ethylhexyl, n-Decyl oder n-Dodecyl sein. Vorzugsweise ist $R^2$ Wasserstoff oder Methyl.

$R^3$ als Alkyl kann z.B. Methyl, Ethyl, Isopropyl, n-Butyl oder n-Hexyl sein.

$R^3$ als Cycloalkyl kann Cyclopentyl oder Cyclohexyl sein.

$Z_1$ ist vorzugsweise eine direkte Bindung oder eine Gruppe $-NHCH_2CH_2-$.

$Z_2$ ist vorzugsweise $-CH_2CH_2-$.

G ist vorzugsweise eine Gruppe der Formel IV.

E als Alkylen kann z.B. 1,2-Bthylen, Tri-, Tetra-, Hexa-, Octa- oder Decamethylen sein. E als Phenylen kann o-, m- oder p-Phenylen sein. Bevorzugt ist E $C_2$-$C_8$-Alkylen oder Phenylen.

L ist bevorzugt $-CH_2CH_2-$, $-CH_2CH(CH_3)-$, $-(CH_2)_3-$ oder $-CH_2-CH(Phenyl)-$.

Der Index n gibt den durchschnittlichen Polymerisationsgrad an. Das Molekulargewicht der oligomeren Polyesteramide, wie es durch Gelpermeationschromatographie bestimmt wird, beträgt etwa 400 bis 10.000.

Die Aminoalkohole der Formel I können allgemein durch Hydroxyalkylierung der entsprechenden primären Amine erhalten werden. Sofern in Formel 1 der Rest G eine Gruppe der Formel IV ist, worin $Z_1$ eine direkte Bindung darstellt, können die Aminoalkohole auch aus den entsprechenden 4-Piperidonen durch reduktive Aminierung erhalten werden:

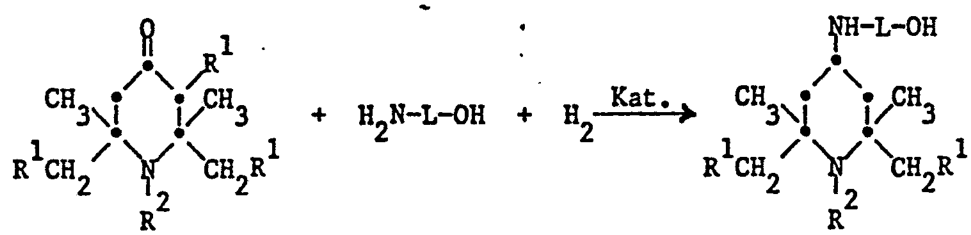


Die Reaktion von I mit II kann lösungsmittelfrei oder in inerten Lösungsmitteln wie z.B. Toluol, Xylol, Mesitylen oder Chlorbenzol ausgeführt werden. Bevorzugt arbeitet man ohne Lösungsmittel bei Temperaturen von etwa 120-200°C. Die Reaktion wird durch basische Katalysatoren wie z.B. Natriumalkoholate, Titantetraalkoxide oder Lithiumamide beschleunigt.

Es war überraschend, dass unter diesen Bedingungen eine Amidbildung eintritt, da es bekannt ist, dass sich zwar primäre Amine mittels Carbonsäure-niederalkylestern glatt acylieren lassen, dass aber eine solche Reaktion mit sekundären Aminen im allgemeinen nur dann möglich ist, wenn der Carbonsäureester aktiviert ist. So führt z.B. die versuchte Umsetzung von 4-Butylamino-2,2,6,6-tetramethylpiperidin mit Capronsäureethylester bei 170°C nicht zur Bildung eines Amides. Im vorliegenden Fall war daher zu erwarten, dass nicht die sekundäre Aminogruppe sondern die Hydroxylgruppe von I acyliert wird. Eine solche O-Acylierung findet auch tatsächlich statt, wenn man versucht, einen Aminoalkohol der Formel I, in dem L ein längerkettiger Alkylenrest, beispielsweise Pentamethylen ist, mit einem Diester II umzusetzen. Es tritt dabei eine Umesterung ein. Nur mit Aminoalkoholen 1, in denen L eine 2- oder 3-gliedrige Kohlenstoff-Kette ist, tritt Polykondensation ein. Möglicherweise spielt hierbei die Ausbildung cyclischer Reaktionskomplexe eine Rolle.

In einer speziellen Ausführungsform kann man die Polykondensation auch mit einem Gemisch zweier Dicarbonsäureester der Formel I durchführen und erhält dann Copolyesteramide der Formel IIIa

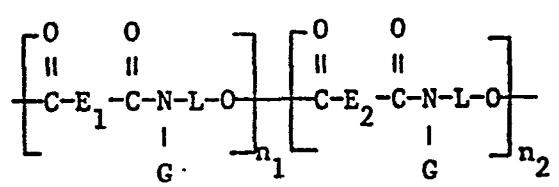 (IIIa),

worin $E_1$ und $E_2$ verschiedene Reste E sind und die Summe von $n_1$ und $n_2$ einen Wert von 2 bis 50, vorzugsweise 2 bis 10, darstellt. Das Verhältnis von $n_1$ zu $n_2$ kann dabei in weitem Bereich variieren, vorzugsweise im Bereich 1:9 bis 9:1.

Die Produkte des erfindungsgemässen Verfahrens sind je nach ihrer Struktur und nach ihrem Molekulargewicht spröde oder weiche Harze oder auch viskose Flüssigkeiten. Man kann die physikalischen Eigenschaften durch Auswahl der Komponenten I und II beeinflussen sowie durch Verwendung zweier verschiedener Dicarbonester II. Das Molekulargewicht lässt sich beeinflussen durch das Molverhältnis von I und II oder durch Zusatz monofunktioneller Verbindungen als Kettenabbrecher.

Unabhängig von ihren physikalischen Eigenschaften sind die oligomeren Polyesteramide als Lichtschutzmittel für organische Materialien verwendbar, die lichtempfindlich sind, vor allem aber als Lichtschutzmittel für organische Polymere. Beispiele für Polymere, die auf diese Weise stabilisiert werden

können, sind die folgenden:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α, β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexa-methylenterephthalamid, Poly-m-phenylenisophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

Von besonderer Bedeutung ist die Stabilisierung von Polyolefinen, Styrolpolymeren, Polyamiden und

Polyurethanen. Beispiele hierfür sind Polyethylen hoher und niedriger Dichte, Polypropylen, Ethylen-Propylen-Copolymere, Polystyrol, Styrol-Butadien-Acrylnitril-Terpolymere, Mischungen von Polyolefinen mit Styrolpolymeren und Polyurethane auf Polyether- oder Polyesterbasis.

Die oligomeren Esteramide werden den Kunststoffen in einer Menge von 0,05 bis 5 Gew.-%, bezogen auf das zu stabilisierende Material, zugesetzt, vorzugsweise verwendet man 0,1 bis 2,5 Gew.-%. Die Einarbeitung kann während oder nach der Herstellung der Polymeren erfolgen, beispielsweise durch Zugabe zu den Polymeren vor oder während deren Fomgebung. Die oligomeren Stabilisatoren können auch in Form eines Masterbatches zu den Kunststoffen zugesetzt werden.

Die Verwendung der oligomeren Polyesteramide als Lichtschutzmittel kann in Kombination mit anderen bekannten Stabilisatoren geschehen, wie z.B. mit anderen Lichtschutzmitteln oder mit Antioxidantien, Metalldesaktivatoren oder anderen Costabilisatoren.

Beispiele hierfür sind die folgenden Stabilisatoren:

**1. Antioxidantien**

**1.1. Alkylierte Monophenole**

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

**1.2. Alkylierte Hydrochinone**

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

**1.3. Hydroxylierte Thiodiphenylether**

2,2'-Thio-bis-(6-tert.butyl-4-methylphenol)
2,2'-Thio-bis-(4-octylphenol)
4,4'-Thio-bia-(6-tert.butyl-3-methylphenol)
4,4'-Thio-bis-(6-tert.butyl-2-methylphenol)

**1.4. Alkyliden-Bisphenole**

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2 2'-Methylen-bis-(4,6-di-tert.butylphenol)
2 2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan

2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

**1.5 Benzylverbindungen**

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

**1.6. Acylaminophenole**

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

**1.7. Ester der β-(3,5-Di-tert.butyl-4-hydroxyphenyl)-propionsäure**

mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyl-oxalsäurediamid

**1.8. Ester der β(5-tert.butyl-4-hydroxy-3-methylphenyl)-propionsäure** mit ein- oder mehrwertigen Alkoholen, wie z.B. mit

Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyl-oxalsäurediamid

**1.9. Amide der β-(3,5-Di-tert.butyl-4-hydroxyphenyl)-propionsäure,**

wie z.B.
N,N'-Di-(3,5-di-tert.butyl-4-hydroxyphenylpropionyl)-hexamethylen-diamin
N,N'-Di-(3,5-di-tert.butyl-4-hydroxyphenylpropionyl)-trimethylen-diamin
N,N'-Di-(3,5-di-tert.butyl-4-hydroxyphenylpropionyl)-hydrazin

## 2. UV-Absorber und Lichtschutzmittel

### 2.1. 2-(2'-Hydroxyphenyl)-benztriazole,

wie z.B. das 5'-Methyl-, 3',5'-Di-tert.butyl-, 5'-Tert.butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

### 2.2. 2-Hydroxybenzophenone,

wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

### 2.3. Ester von gegebenenfalls substituierten Benzoesäuren,

wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexa-decylester.

### 2.4. Acrylate,

wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

### 2.5. Nickelverbindungen,

wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyldiethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

### 2.6. Sterisch gehinderte Amine,

wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzyl-malonsäure-bis (1,2,2,6,6-pentamethylpiperidyl)-ester, Kondensationsprodukt aus 1-Hydroxy-ethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat, Tetrakis-(2,2,6,6-tetramethyl-4-piperidyl)-1,2,3,4-butantetracarbonsäure, 1,1'-(1,2-ethandiyl)-bis-(3,3,5,5-tetramethyl-piperazinon).

### 2.7. Oxalsäurediamide,

wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.bu-tyl-oxanilid, Gemische von ortho- und para-Methoxy- sowie von o- und p-Ethoxy-di-substituierte Oxaniliden.

**3. Metalldesaktivatoren,**

wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzylidenoxalsäuredihydrazid.

**4. Phosphite und Phosphonite,**

wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythrit-diphosphit, Tristearylsorbit-triphosphit, Tetrakis-(2,4-di-tert.butylphenyl)-4,4'-biphenylen-diphosphonit.

**5. Peroxidzerstörende Verbindungen,**

wie z.B. Ester der β-Thio-dipropionsäure beispielsweise der Lauryl- Stearyl-, Myristyl- oder Tridecylester Mercaptobenzimidazol das Zinksalz des 2-Mercapto-benzimidazols, Zink-dibutyl-dithiocarbamat, Dioctadecyldisulfid, Pentaerythrit-tetrakis-(β-dodecylmercapto)-propionat.

**6. Polyamidstabilisatoren,**

wie z.B. Kupfersalze in Kombination mit Jodiden und/oder Phosphorverbindungen und Salze des zweiwertigen Mangans.

**7. Basische Co-Stabilisatoren,**

wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

**8. Nukleierungsmittel,**

wie z.B. 4-tert.Butylbenzoesäure, Adipinsäure, Diphenylessigsäure.

**9. Füllstoffe und Verstärkungsmittel,**

wie z.B. Calciumcarbonat, Silikate, Glasfasern, Asbest, Talk, Kaolin, Glimmer, Bariumsulfat, Metalloxide und -hydroxide, Russ, Graphit.

**10. Sonstige Zusätze,**

wie z.B. Weichmacher, Gleitmittel, Emulgatoren, Pigmente, Optische Aufheller, Flammschutzmittel, Antistatika, Treibmittel.

Bei der Mitverwendung solcher bekannter Stabilisatoren können synergistische Effekte auftreten, was besonders bei der Mitverwendung von UV-Absorbern häufig der Fall ist.

Zusammen mit solchen Stabilisatoren können auch andere in der Kunststoff-Technologie übliche Zusätze zugegeben werden, wie z.B. Pigmente, Farbstoffe, Füllstoffe, Russ, Asbest, Glasfasern, Flammschutzmittel oder Antistatika.

Die erfindungsgemäss herstellbaren Oligomeren können auch als Mittel zur Erhöhung der Anfärbbarkeit von synthetischen Fasern mit Säurefarbstoffen verwendet werden. Ein solcher Effekt ist vor allem bei Polyolefinen wie z.B. bei Polypropylenfasern von Bedeutung.

Wie eingangs erwähnt sind einige der erfindungsgemäss herstellbaren Oligomeren in der DE-OS 31 11 739 allgemein beschrieben. Die übrigen Produkte sind jedoch neue Verbindungen. Es handelt sich dabei insbesondere um die Verbindungen der Formel III und IIIa, in denen E, bzw. $E_1$ und $E_2$ eine Gruppe $-(CH_2)_x-$ mit $x = 4-12$ oder eine meta- oder para-Phenylengruppe darstellen.

Die folgenden Beispiele erläutern die Erfindung näher ohne sie auf den Umfang der Beispiele zu begrenzen. Die Temperaturen sind darin als °C angegeben.

**Beispiel 1:**

Eine Mischung von 8,71 g (0,05 Mol) 2-(2,2,6,6-Tetra-methyl-4-piperidylamino)-ethanol und 10,02 g (0,05 Mol) Dimethyladipat wird 2 h auf 120° erwärmt. Nach Zugabe von 0,2 g Titan-IV-butoxid wird die Temperatur auf 160° gesteigert unter Abdestillieren des gebildeten Methanols. Das Reaktionsgemisch wird weitere 8 h bei 190° gerührt bis 1,2 ml Methanol erhalten werden. Das Produkt wird in 100 ml Tetrahydrofuran gelöst und durch Zugabe von Hexan gefällt. Das so erhaltene Polyesteramid wird im Vakuum (0,1 mm Hg (0,13 mbar)) 15 h getrocknet. Man erhält 11,7 g eines halbfesten Harzes mit einem durchschnittlichen Molekulargewicht (bestimmt durch Gelpermeationschromatographie = GPC) von etwa 1000.

**Beispiel 2:**

Wenn man 25,0 g (0,125 Mol) 2-(2,2,6,6-Tetramethyl-4-piperidylamino)-ethanol mit 14,64 g (0,10 Mol) Dimethylsuccinat und 4,36 g (0,025 Mol) Dimethyladipat wie im Beispiel 1 beschrieben umsetzt, so erhält man 26,0 g eines festen Copolymeren, das bei 45-50° schmilzt.

**Beispiel 3:**

Wenn man 42,0 g (0,20 Mol) 3-(2,2,6,6-Tetramethyl-4-piperidylamino)-propanol-1 mit 34,8 g (0,20 Mol) Dimethyladipat wie im Beispiel 1 beschrieben umsetzt, so erhält man 48,6 g eines gelblichen Harzes. Das durchschnittliche Molekulargewicht beträgt etwa 700 (GPC).

**Beispiel 4:**

Wenn man 55,0 g (0,257 Mol) 1-(2,2,6,6-Tetramethyl-4-piperidylamino)-propanol-2 mit 37,5 g (0,257 Mol) Dimethylsuccinat wie im Beispiel 1 beschrieben umsetzt, so erhält man 35,0 g eines gelblichen Harzes vom Smp. 49-54°.

**Beispiel 5:**

Wenn man 53,6 g (0,25 Mol) 1-(2,2,6,6-Tetramethyl-4-piperidylamino)-propanol-2 mit 43,5 g (0,35 Mol) Dimethyladipat wie in Beispiel 1 beschrieben umsetzt, erhält man 69.6 g einer viskosen Masse. Das durch GPC bestimmte Molekulargewichtsmittel beträgt etwa 800.

**Beispiel 6:**

21,44 g (0,10 Mol) 1-(2,2,6,6-Tetramethyl-4-piperidyl-amino)-propanol-2 und 19,50 g (0,10 Mol) Dimethylphthalat werden 1 h auf 120° erhitzt. Nach Zugabe von 0,7 g Titan-IV-butoxid erhitzt man weitere 4 h auf 170°, wobei 5,4 ml Methanol abgespalten werden. Dann werden 100 ml Tetrahydrofuran zugegeben und die Mischung 2 h bei 60° gerührt. Der dabei erhaltene weisse Feststoff wird abfiltriert und mit Hexan gewaschen. Man erhält 9,4 g Produkt das bei 135° erweicht und bei 250-280° schmilzt. Das mittels GPC ermittelte Molekulargewicht beträgt etwa 700.

**Beispiel 7:**

Aus 21,44 g (0,10 Mol) 1-(2,2,6,6-Tetramethyl-4-piperidyl-amino)-propanol-2 und 19,50 g (0,10 Mol) Dimethylterephthalat erhält man unter den Bedingungen des Beispiels 6 bei Fällung mit Hexan aus Tetrahydrofuranlösung 15,4 g weissen Feststoff, der bei 61-72° schmilzt. Das Molekulargewichtsmittel (GPC) beträgt 850.

**Beispiel 8:**

Aus 21,44 g (0,10 Mol) 1-(2,2,6,6-Tetramethyl-4-piperidylamino)-propanol-2 und 19.50 g (0,10 Mol) Dimethylisophthalat erhält man unter den Bedingungen des Beispiels 6 21 g eines klebrigen Feststoffes, der bei 50-60° schmilzt und ein mittleres Molekulargewicht von 850 hat (GPC).

**Beispiel 9:**

Bei Umsetzung von 24,34 g (0,10 Mol) N-(2,2,6,6-Tetra-methyl-4-piperidyl)-N'-(2-hydroxyethyl)-ethylendiamin mit 14,61 g (0,10 Mol) Dimethylsuccinat gemäss Beispiel 1 erhält man 18 g eines gelblichen Feststoffes, der bei 130-150° schmilzt. Das durch GPC bestimmte mittlere Molekulargewicht beträgt 1900.

**Beispiel 10:**

Bei Umsetzung von 41,60 g (0,171 Mol) N-(2,2,6,6-Tetra-methyl-4-piperidyl)-N'-(2-hydroxyethyl)-ethylendiamin mit 29,68 g (0,171 Mol) Dimethyladipat gemäss Beispiel 1 erhält man 57,5 g eines niedrig schmelzenden Harzes, dessen Molekulargewichtsmittel etwa 1000 beträgt (GPC).

**Beispiel 11:**

Bei Umsetzung von 24,34 g (0,10 Mol) N-(2,2,6,6-Tetra-methyl-4-piperidyl)-N'-(2-hydroxyethyl)-ethylendiamin mit 20,23 g (0,10 Mol) Dimethylsuberat gemäss Beispiel 1 erhält man 17 g eines gelblichen Harzes.

**Beispiel 12:**

Eine Lösung von 24,34 g (0,10 Mol) N-(2,2,6,6-Tetra-methyl-4-piperidyl)-N'-(2-hydroxyethyl)-ethylendiamin und 19,42 g (0,10 Mol) Dimethylphthalat in 140 ml Xylol wird 2 h zum Sieden erhitzt unter Abdestillieren von 70 ml Xylol. Nach Zugabe von 0,5 g Titan-IV-butoxid wird die Temperatur auf 160° gesteigert, wobei das entstandene Methanol langsam abdestilliert. Zum Schluss wird 8 h bei 190° gerührt.

Das Produkt wird in 100 ml Tetrahydrofuran gelöst und durch Zugabe von Hexan gefällt. Man erhält 11.7 g eines halb-festen Materials, das ein durchschnittliches Molekulargewicht von etwa 1000 besitzt (GPC).

**Beispiel 13:**

Bei Umsetzung von 12,20 g (0,05 Mol) N-(2,2,6,6-Tetra-methyl-4-piperidyl)-N'-(2-hydroxyethyl)-ethylendiamin mit 9,70 g (0,05 Mol) Dimethylterephthalat gemäss Beispiel 1 wird ein festes Produkt erhalten. Dieses wird in 22 ml Ethanol gelöst und durch Zugabe von 30 ml Xylol ausgefällt. Man erhält 12 g eines bräunlichen Feststoffes vom Smp. 190-210°, der ein mittleres Molekulargewicht von 950 besitzt (GPC).

**Beispiel 14:**

Polypropylen (Hercules Profax®6501), das 0,1 Gew.-% Calciumstearat, aber kein Antioxydans enthält, wird mit 0,1 Gew.-% eines erfindungsgemäss herstellbaren Lichtschutzmittels vermischt. Die Mischung wird granuliert und in einem Extruder mit einer 10 cm breiten Filmdüse zu einem Band von 0,12 mm Stärke

verarbeitet. Das Band wird zu 6,4 mm breiten Streifen zerschnitten, welche bei 107° suf die sechsfache Lauge verstreckt werden. Die erhaltenen Filmbändchen haben eine Stärke von 0,05 mm.

Die Bändchen werden in einem Kohlebogen-Weatherometer (unter Befeuchtung) belichtet. Von Zeit zu Zeit wird die Zugfestigkeit der belichteten Bändchen gemessen. Die Belichtungszeit bis zu 50% der ursprünglichen Reissfestigkeit ist ein Kriterium für die Wirksamkeit des verwendeten Lichtschutzmittels und ist in der folgenden Tabelle aufgeführt.

| **Lichtschutzmittel Produkt des Beispiels** | **Belichtungszeit (h) bis 50% Reissfestigkeit** |
|---|---|
| 1 | 2850 |
| 2 | 3250 |
| 9 | 2575 |
| 10 | 3080 |
| 11 | 2530 |
| 13 | 1070 |
| ohne Lichtschutzmittel | 385 |

**Patentansprüche**

1. Verfahren zur Herstellung von oligomeren Polyesteramiden der Formel III

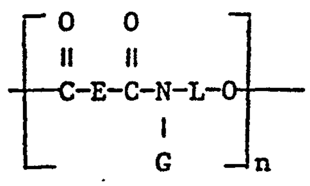

(III)

worin G eine Gruppe der Formel IV oder V bedeutet,

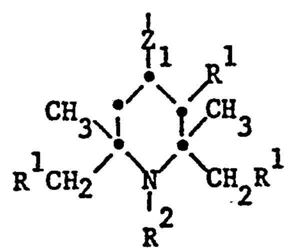

(IV)

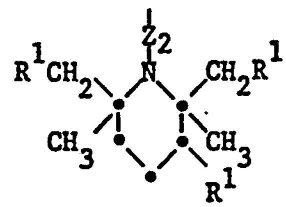

(V)

worin $R^1$ Wasserstoff oder $C_1$-$C_4$-Alkyl ist,
$R^2$ Wasserstoff, $C_1$-$C_{12}$-Alkyl, Allyl, Benzyl, Acetyl oder Acryloyl ist,
$Z_1$ eine direkte Bindung oder eine Gruppe $-NHCH_2CH_2-$, $-NH-(CH_2)_3-$ oder $-O-CH_2CH_2-$, deren Heteroatom an den Piperidinring gebunden ist, darstellt und
$Z_2$ $-(CH_2)_2-$ oder $-(CH_2)_3-$ darstellt,
E $C_2$-$C_{12}$-Alkylen, Phenylen, 5-Norbornen-2,3-diyl oder -CH=CH- bedeutet und
L $-(CH_2)-$, $(CH_2)_3-$ oder $-CH_2-CH(R^3)-$ bedeutet, worin
$R^3$ $C_1$-$C_6$-Alkyl, $C_5$-$C_6$-Cycloalkyl oder Phenyl ist, und
n einen Wert von 2 bis 50, vorzugsweise von 2 bis 10, darstellt,
durch Umsetzung eines Aminoalkohols der Formel 1

G - NH - L - OH (I)

mit einem Dicarbonsäureester der Formel II

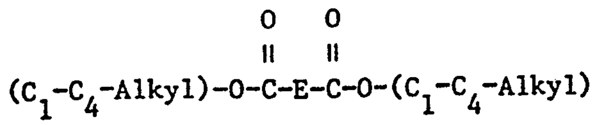

(II)

im annähernden Molverhältnis 1:1.

2. Verfahren gemäss Anspruch 1, dadurch gekennzeichnet, dass man die Umsetzung lösungsmittelfrei bei einer Temperatur von 120-200° C in Gegenwart eines basischen Katalysators durchführt.

3. Verfahren gemäss Anspruch 1, dadurch gekennzeichnet, dass man einen Aminoalkohol der Formel I verwendet, worin G eine Gruppe der Formel IV ist, in der $R^1$ Wasserstoff ist, $R^2$ Wasserstoff oder Methyl ist und $Z_1$ eine direkte Bindung oder eine Gruppe $-NHCH_2CH_2-$ ist, E $C_2$-$C_8$-Alkylen oder Phenylen ist und L $-CH_2CH_2-$, $(CH_2)_3-$, $-CH_2CH(CH_3)-$ oder $-CH_2-CH(Phenyl)-$ ist.

4. Verfahren gemäss Anspruch 1, dadurch gekennzeichnet, dass man zwei verschiedene Dicarbonsäureester verwendet und dadurch ein oligomeres Copolyesteramid der Formel IIIa erhält

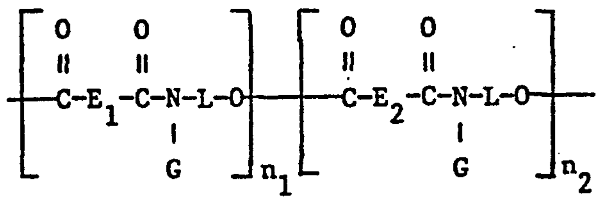

(IIIa)

worin $E_1$ und $E_2$ untereinander verschiedene Reste mit der Bedeutung von E sind und die Summe von $n_1$ und $n_2$ einen Wert von 2 bis 50, vorzugsweise von 2 bis 10, darstellt.

5. Verwendung der gemäss den Ansprüchen 1 oder 4 hergestellten Produkte als Lichtschutzmittel für organische Materialien, insbesondere für organische Polymere.

6. Oligomere Polyesteramide der Formel III des Anspruches 1 oder der Formel IIIa des Anspruches 4, worin E, $E_1$ und $E_2$ eine Gruppe $-(CH_2)_x$-mit $x = 4$-12 oder meta- oder para-Phenylen darstellen, G, L und n die in Anspruch 1 und $n_1$ und $n_2$ die in Anspruch 4 gegebene Bedeutung haben.

## Claims

1. A process for the preparation of an oligomeric polyester amide of formula III

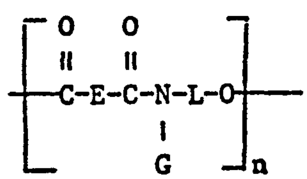

(III)

wherein G is a group of formula IV or V

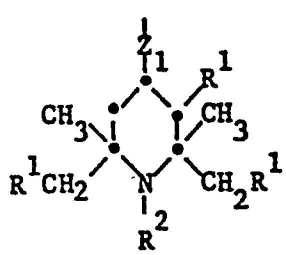

(IV)

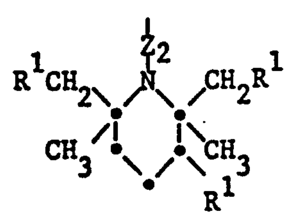

(V)

wherein

$R^1$ is hydrogen or $C_1$-$C_4$alkyl,

$R^2$ is hydrogen, $C_1$-$C_{12}$alkyl, allyl, benzyl, acetyl or acryloyl,

$Z_1$ is a direct bond or a group selected from $-NHCH_2CH_2-$, $-NH-(CH_2)_3-$ or $-O-CH_2CH_2-$, with the hetero atom

being attached to the piperidine ring, and
$Z_2$ is $-(CH_2)_2-$ or $-(CH_2)_3$,
E is $C_2$-$C_{12}$alkylene, phenylene, 5-norbornen-2,3-diyl or $-CH=CH-$, and
L is $-(CH_2)_2-$, $-(CH_2)_3-$ or $-CH_2-CH(R^3)-$, wherein
$R^3$ is $C_1$-$C_6$alkyl, $C_5$-$C_6$cycloalkyl or phenyl,
and
n is a value from 2 to 50, preferably from 2 to 10,
which process comprises reacting an aminoalcohol of formula I

$$G-NH-L-OH \qquad (I)$$

with a dicarboxylate of formula II

$$(C_1\text{-}C_4\text{-alkyl})-O-\overset{\overset{O}{\|}}{C}-E-\overset{\overset{O}{\|}}{C}-O-(C_1\text{-}C_4\text{-alkyl}) \qquad (II)$$

in the approximate molar ratio of 1:1.

2. A process according to claim 1, wherein the reaction is carried out without a solvent in the temperature range from 120°-200° C, in the presence of a basic catalyst.

3. A process according to claim 1, which comprises the use of an aminoalcohol of formula I, wherein G is a group of formula IV in which $R^1$ is hydrogen, $R^2$ is hydrogen or methyl and $Z_1$ is a direct bond or a $-NHCH_2CH_2-$ group, E is $C_2$-$C_8$alkylene or phenylene, and L is $-CH_2CH_2-$, $-(CH_2)_3-$, $-CH_2CH(CH_3)-$ or $-CH_2-CH(phenyl)-$.

4. A process according to claim 1, wherein two different dicarboxylates are used to give an oligomeric copolyester amide of formula IIIa

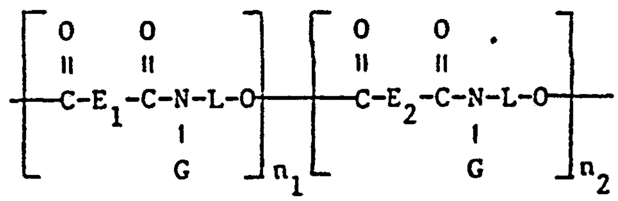

(IIIa)

wherein $E_1$ and $E_2$ are independently different radicals having the meaning of E and the sum of $n_1 + n_2$ is a value from 2 to 50 preferably from 2 to 10.

5. Use of a product prepared according to either claim 1 or claim 4 as light stabiliser for organic materials, in particular for organic polymers.

6. An oligomeric polyester amide of formula III according to claim 1 or of formula IIIa according to claim 4, wherein E, $E_1$ and $E_2$ are a $-(CH_2)$ - group, in which x is a value from 4 to 12, or m- or p-phenylene, G, L and n are as defined in claim 1 and $n_1$ and $n_2$ are as defined in claim 4.

**Revendications**

1.- Procédé de préparation de polyesteramides oligomères répondant à la formule III:

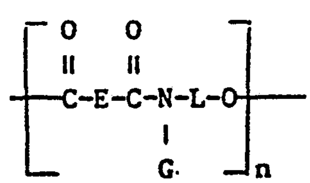

(III)

dans laquelle
G représente un radical répondant à l'une des formules IV et V:

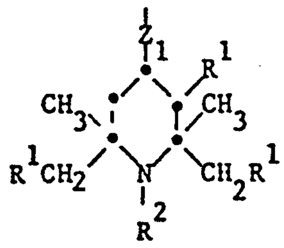 (IV) 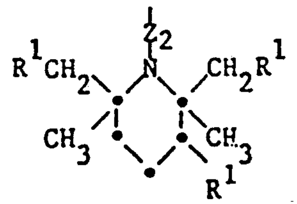 (V)

dans lesquelles

$R^1$ représente l'hydrogène ou un alkyle en $C_1$-$C_4$,

$R^2$ représente l'hydrogène, un alkyle en $C_1$-$C_{12}$, un allyle, un benzyle, un acétyle ou un acryloyle,

$Z_1$ représente une liaison directe ou un radical $-NHCH_2CH_2-$, $-NH-(CH_2)_3-$ ou $-O-CH_2CH_2-$ dont l'hérétoatome est relié au noyau pipéridinique, et

$Z_2$ représente $-(CH_2)_2-$ ou $-(CH_2)_3-$,

E représente un alkylène en $C_2$-$C_{12}$, un phénylène, un norbornène-5 -diyle-2,3 ou un radical -CH=CH-,

L représente $-(CH_2)_2-$, $-(CH_2)_3-$ ou $-CH_2-CH(R^3)-$, le symbole $R^3$ désignant un alkyle en $C_1$-$C_6$, un cycloalkyle en $C_5$-$C_6$ ou un phényle,

et

n représente un nombre de 2 à 50, de préférence de 2 à 10,

par réaction d'un amino-alcool de formule I:

$$G - NH - L - OH \qquad (I)$$

avec un diester d'acide dicarboxylique de formule II:

$$(C_1\text{-}C_4\text{-Alkyl})\text{-}O\text{-}\overset{O}{\overset{\|}{C}}\text{-}E\text{-}\overset{O}{\overset{\|}{C}}\text{-}O\text{-}(C_1\text{-}C_4\text{-Alkyl}) \qquad (II)$$

dans un rapport molaire à peu près égal à 1:1.

2.- Procédé selon la revendication 1, caractérisé en ce qu'on effectue la réaction sans solvant à une température de 120 à 200°C, en présence d'un catalyseur basique.

3.- Procédé selon la revendication 1, caractérisé en ce qu'on utilise un amino-alcool de formule I dans lequel G représente un radical de formule IV dans lequel $R^1$ désigne l'hydrogène, $R^2$ l'hydrogène ou un méthyle et $Z_1$ une liaison directe ou un radical $-NHCH_2CH_2-$, E représente un alkylène en $C_2$-$C_8$ ou un phénylène, et L un radical $-CH_2CH_2-$, $-(CH_2)_3-$, $-CH_2CH(CH_3)-$ ou $-CH_2-CH(\text{phényl})-$.

4.- Procédé selon la revendication 1, caractérisé en ce qu'on utilise deux diesters d'acides dicarboxyliques différents et en ce que l'on obtient ainsi un copolyester amide oligomère répondant à la formule IIIa:

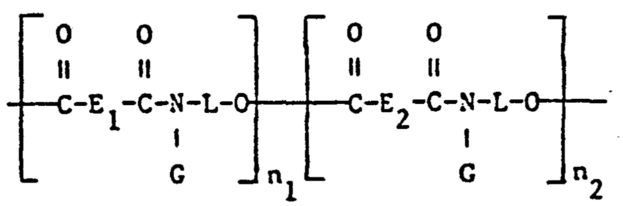 (IIIa)

dans laquelle $E_1$ et $E_2$ sont des radicaux différents l'un de l'autre qui ont chacun la signification qui a été donnée pour E, et la somme $(n_1 + n_2)$ a une valeur de 2 à 50, de préférence de 2 à 10.

5.- Application des produits préparés selon l'une quelconque des revendications 1 à 4, comme stabilisants à la lumière pour des matières organiques, plus spécialement, pour des polymères organiques.

6.- Polyesteramides oligomères qui repondent à la formule III représentée à la revendication 1 ou la formule IIIa représentée à la revendication 4, dans lesquels E, $E_1$ et $E_2$ représentent chacun un radical $-(CH_2)_x-$ dont l'indice x est un nombre de 4 à 12, ou un radical m-phénylène ou p-phénylène, G, L et n ont les significations données à la revendication 1, et $n_1$ et $n_2$ ont les signfications données à la revendication 4.